(19) Europäisches Patentamt
European Patent Office
Office européen des brevets

(11) Veröffentlichungsnummer: **0 024 651**
**B1**

(12) # EUROPÄISCHE PATENTSCHRIFT

(45) Veröffentlichungstag der Patentschrift: **29.12.82**

(21) Anmeldenummer: **80104833.1**

(22) Anmeldetag: **14.08.80**

(51) Int. Cl.³: **C 07 C 27/04,** B 01 J 31/28, C 07 C 45/62, C 07 C 29/17, C 07 C 47/21, C 07 C 49/203, C 07 C 33/025, C 07 C 33/02

(54) **Verfahren zur Herstellung von olefinisch ungesättigten Carbonylverbindungen und Alkoholen.**

(30) Priorität: **24.08.79 DE 2934250**

(43) Veröffentlichungstag der Anmeldung: **11.03.81 Patentblatt 81/10**

(45) Bekanntmachung des Hinweises auf die Patenterteilung: **29.12.82 Patentblatt 82/52**

(84) Benannte Vertragsstaaten: **BE CH DE FR GB IT LI NL**

(56) Entgegenhaltungen:
**EP-A-0 008 741**
**BE-A-830 446**
**DE-A-2 357 645**
**FR-A-474 809**
**FR-A-1 399 393**
**FR-A-2 087 835**
**FR-A-2 405 232**
**US-A-3 055 840**
**CHEMICAL ABSTRACTS, Band 90, Nr. 19, 7. Mai 1979, Seite 633, Zusammenfassung Nr. 152369y, Columbus, Ohio, US, D. V. SOKOL'SKII et al.: »Hydrogenation of citral to citronellol on group VIII metals«**
**CHEMICAL ABSTRACTS, Band 84, Nr. 23, 7. Juni 1976, Seite 492, Zusammenfassung Nr. 165043p, Columbus, Ohio, US, D. V. SOKOL'SKII et al.: »On the hydrogenation of unsaturated aldehydes«**

(73) Patentinhaber: **BASF Aktiengesellschaft, Carl-Bosch-Strasse 38, D-6700 Ludwigshafen (DE)**

(72) Erfinder: **Horner, Michael, Dr., Pfalzgrafenstrasse 15B, D-6730 Neustadt (DE)**
Erfinder: **Irgang, Matthias,Dr., Gontardstrasse 4, D-6800 Mannheim 1 (DE)**
Erfinder: **Nissen, Axel, Dr., Panoramastrasse 51, D-6906 Leimen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von olefinisch ungesättigten Carbonylverbindungen und Alkoholen

Die vorliegende Erfindung betrifft ein verbessertes Verfahren zur Herstellung von olefinisch ungesättigten Carbonylverbindungen und Alkoholen der allgemeinen Formeln I und II

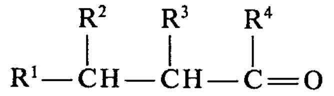

I

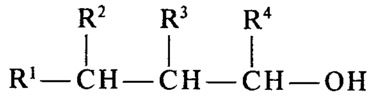

II

in denen $R^1$ einen olefinisch ungesättigten organischen Rest und $R^2$, $R^3$ und $R^4$ Wasserstoff oder eine $C_1$- bis $C_4$-Alkylgruppe bedeuten, durch Hydrierung von $\alpha,\beta$-ungesättigten Carbonylverbindungen der allgemeinen Formel III

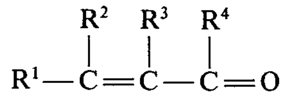

III

mit Wasserstoff in Gegenwart von Edelmetallkatalysatoren in der Flüssigphase. Speziell betrifft die Erfindung die Hydrierung von Citral (III a)

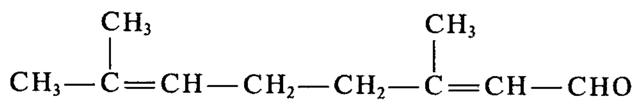

III a

zu Citronellal (I a)

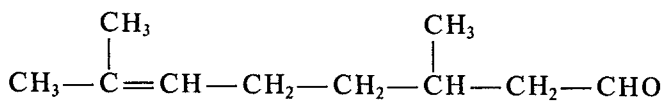

I a

bzw. zu Citronellol (II a)

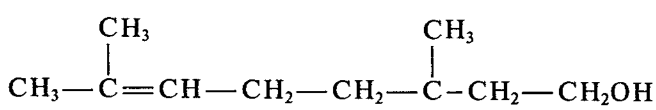

II a

Es ist allgemein bekannt und bedarf deshalb keiner näheren Erläuterung, daß man die Carbonylgruppe von $\alpha,\beta$-ungesättigten Carbonylverbindungen selektiv oder weitgehend selektiv, d. h. ohne daß die olefinische Doppelbindung angegriffen wird, mittels Ruthenium-, Rhodium-, Osmium-, Iridium- oder Platinkatalysatoren zu der entsprechenden Alkoholgruppe hydrieren kann, wobei auch sonstige olefinische Doppelbindungen außer der besonders empfindlichen $\alpha,\beta$-Doppelbindung mehr oder weniger intakt bleiben. Ein besonders wirtschaftliches Verfahren dieser Art, welches durch die Mitverwendung eines tertiären Amins bei der Hydrierung gekennzeichnet ist, ist Gegenstand der prioritätsgleichen europäischen Patentanmeldung EP-OS 0 024 648.

Verwendet man anstelle der genannten Hydrierkatalysatoren Palladium, so werden, wie ebenfalls allgemein bekannt ist, vornehmlich die olefinischen Doppelbindungen hydriert, wogegen die Carbonylgruppe nicht oder nur in geringerem Maße angegriffen wird. Besonders gute Ergebnisse erzielt man hierbei nach dem Verfahren der älteren europäischen Patentanmeldung EP-OS 0 008 741, welches durch die Mitverwendung von 15 bis 50 Gew.-%, bezogen auf die Menge der eingesetzten Carbonylverbindung, eines tertiären Amins bei der Hydrierung gekennzeichnet ist.

Möchte man nach den Verfahren des Standes der Technik, ausgehend von den Carbonylverbindungen III, zu den Carbonylverbindungen I und den Alkoholen II gelangen, so müßte man zwei getrennte Hydrierungen, die einerseits in bezug auf die Carbonylgruppe und andererseits in bezug auf die $\alpha,\beta$-ungesättigte olefinische Doppelbindung selektiv sind, hintereinanderschalten. Da hierin ein offensichtlicher verfahrenstechnischer Nachteil liegt, wurden bereits mehrere Verfahren zur gleichzeitigen Hydrierung vorgeschlagen.

Verwendet man hierzu nach der Methode von Research of India, Bd. 17/1 (1972), Seite 11 einen Cu/Cr-Katalysator, so erzielt man bei der Hydrierung von Citral lediglich Citronellol-Ausbeuten von 70%. Mit Raney-Nickel (US-PS 3 860 657 und DE-OS 2 706 862) verläuft die Hydrierung von Citral zu Citronellol zu langsam und zu wenig selektiv. Die nach dem Verfahren der DE-OS 2 706 862 bei der Hydrierung von Citral zu Citronellol mittels chromaktiviertem Raney-Nickel erhältlichen guten

Ausbeuten von bis zu 94% müssen mit unwirtschaftlich langen Reaktionszeiten und großen Katalysatormengen erkauft werden, so daß auch dieses Verfahren für Technik nicht attraktiv ist.

Der Erfindung lag daher vor allem ein verbessertes Verfahren zur gleichzeitigen Hydrierung der Doppelbindungen in der

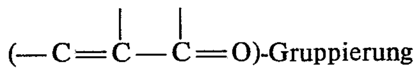

der Carbonylverbindungen III als Aufgabe zugrunde.

Weiterhin war es Aufgabe der Erfindung, die Carbonylverbindungen III nicht nur zu den Alkoholen II, sondern wahlweise auch zu der Zwischenstufe, den $\alpha,\beta$-gesättigten Carbonylverbindungen I zu hydrieren, und zwar mit Hilfe des gleichen Katalysatorsystems und unter nur einfacher Abwandlung der Reaktionsbedingungen. Speziell war es Aufgabe der Erfindung, die technisch besonders wichtige Hydrierung des Citrals IIIa zu den vielseitigen Duftstoffen Citronellal Ia und Citronellol IIa zu verbessern und verfahrenstechnisch einfacher zu gestalten.

Es wurde gefunden, daß man olefinisch ungesättigte Carbonylverbindungen und Alkohole der allgemeinen Formeln I und II

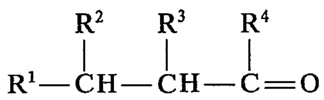 I

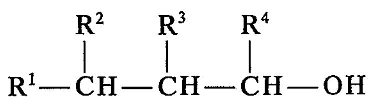 II

in denen $R^1$ einen olefinisch ungesättigten organischen Rest und $R^2$, $R^3$ und $R^4$ Wasserstoff oder eine $C_1$- bis $C_4$-Alkylgruppe bedeuten, durch Hydrierung von $\alpha,\beta$-ungesättigten Carbonylverbindungen III

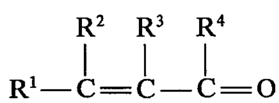 III

mit Wasserstoff in Gegenwart von Edelmetallkatalysatoren in der Flüssigphase auf verfahrenstechnisch und wirtschaftlich vorteilhafte Weise erhält, wenn man die Hydrierung in Gegenwart

- eines Katalysatorsystems aus Palladium einerseits und Ruthenium, Rhodium, Osmium, Iridium oder Platin als aktiven Bestandteilen andererseits sowie
- von 5 bis 40 Gew.-%, bezogen auf die Menge von III, eines tertiären Amins

vornimmt, und zwar

- zur Herstellung von überwiegenden Mengen der Verbindungen I unter einem Wasserstoffdruck von 1 bis etwa 20 bar und
- zur Herstellung von überwiegenden Mengen der Verbindungen II unter einem Wasserstoffdruck von etwa 20 bis 150 bar.

Wesentlich für dieses Verfahren ist es, daß wegen der Mitverwendung der tertiären Amine die olefinische Doppelbindung im Rest $R^1$ praktisch nicht angegriffen wird.

Prinzipiell sind nach den bisherigen Beobachtungen jegliche tertiäre Amine geeignet, so daß es auf deren chemische Natur, sofern sie aufgrund funktioneller Gruppen nicht anderweitig mit den Reaktionspartnern reagieren können, nicht ankommt. Genannt seien beispielsweise

- aliphatische tertiäre Amine mit insgesamt 3 bis 30 C-Atomen, wie vor allem Trimethylamin sowie Triethylamin, Triethanolamin und Trihexylamin,
- cyclische tertiäre Amine wie N-Methylpiperidin, N-Methylmorpholin und N,N′-Dimethylpiperazin
- aliphatisch-cycloaliphatische tertiäre Amine wie N,N-Dimethylcyclohexylamin
- aliphatisch-araliphatische tertiäre Amine wie N,N-Dimethylbenzylamin
- aliphatisch-aromatische tertiäre Amine wie N,N-Dimethylanilin sowie
- heterocyclisch-aromatische tertiäre tertiäre Amine wie Pyridin und Chinolin.

Aus wirtschaftlichen Gründen wird man im übrigen möglichst billige Amine verwenden, deren Siedepunkt entweder deutlich tiefer oder deutlich höher liegt als der des Verfahrensproduktes, da sich in diesen Fällen entweder die Amine oder die Verfahrensprodukte leicht aus dem Reaktionsgemisch abdestillieren lassen.

Die Menge der Amine beträgt vorzugsweise 10 bis 30 Gew.-% des Einsatzstoffes III.

Die für die Hydrierung erforderlichen Kombinationskatalysatoren bestehen aus den Komponenten Pd einerseits und Ru, Rh, Os, Ir oder Pt andererseits. Man kann diese Komponenten in getrennter Form als Substanz- oder Trägerkatalysatoren oder gemeinsam als Kombinationsträgerkatalysatoren einsetzen. Bevorzugt wird die Verwendung in Form getrennter Trägerkatalysatoren, da diese handelsüblich sind.

Nach den bisherigen Beobachtungen eignen sich sämtliche handelsüblichen Katalysatoren gleichermaßen gut, so daß die Betriebsbedingungen bei Wechsel der Katalysatorart entweder gar nicht oder nur geringfügig geändert zu werden brauchen.

Bezogen auf den Metallgehalt und auf die Menge der Ausgangsverbindungen III setzt man das Pd vorzugsweise in Mengen von 0,001 bis 0,1, besonders 0,005 bis 0,1 und die übrigen Platinmetalle in Mengen von 0,002 bis 0,2, besonders 0,01 bis 0,2 Gew.-% ein. Das Gewichtsverhältnis von Pd zu den übrigen Edelmetallen beträgt vorteilhaft 1 : 2 bis 1 : 10. Außerhalb dieses Verhältnisses laufen die beiden Hydrierreaktionen (Hydrierung der Carbonylfuntkion und der $\alpha,\beta$-Doppelbindung) mit merklich unterschiedlichen Geschwindigkeiten ab, so daß man bei vorzeitigem Abbruch der Hydrierung eines der partiell hydrierten Produkte jeweils im Überschuß erhält, ein Effekt, der u. U. erwünscht sein kann, wenn man mehrere Hydrierungsprodukte im bestimmten Mengenverhältnis gleichzeitig herstellen will, z. B. Geraniol oder Nerol oder Citronellal neben Citronellol bei der Hydrierung von Citral. Da die meisten handelsüblichen Katalysatoren Trägerkatalysatoren mit Aktivkohle als Träger und 5 Gew.-% Metallgehalt sind, werden derartige Katalysatoren aus wirtschaftlichen Gründen bevorzugt.

Es empfiehlt sich, die Reaktion in Gegenwart eines Lösungsmittels vorzunehmen. Die Mengen an Lösungsmittel liegen im allgemeinen zwischen 10 und 300, vorzugsweise 25 und 150 Gew.-% von III. Als Lösungsmittel eigenen sich alle inerten Flüssigkeiten, in denen sowohl I, II und III als auch das mitverwendete tertiäre Amin löslich sind. In Betracht kommen beispielsweise die tertiären Amine selber sowie zusätzlich Alkohole wie Methanol und Ethanol, Ether, Aceton und unter den Reaktionsbedingungen flüssige Kohlenwasserstoffe wie Hexan und Cyclohexan. Bevorzugt wird Methanol, und zwar vor allem, wenn man Trimethylamin als Base verwendet, da sich in diesem Falle die Aufarbeitung der Reaktionsgemische besonders einfach gestaltet.

Allgemein nimmt man die Hydrierung bei 20 bis 150° C vor. Möchte man die Carbonylverbindungen I herstellen, so arbeitet man unter einem Wasserstoffdruck von 1 bis etwa 20, vorzugsweise 10 bar, und sind die Alkohole II das Verfahrensziel, so liegt der hierfür geeignete Druck zwischen etwa 20 und 200 bar. Wählt man einen Druck zwischen 10 und 20 bar, oder bricht man die Reaktion vorzeitig ab, so erhält man beide Verfahrensprodukte nebeneinander, was u. U. erwünscht sein kann. Hervorzuheben ist, daß die Bildung von I oder II bei sonst gleichen Reaktionsbedingungen wie Temperatur und Art und Mengenverhältnisse des Katalysatorsystems im wesentlichen nur vom Wasserstoffdruck abhängt, d. h. man hat es mittels der einfachen verfahrenstechnischen Änderung des Druckes in der Hand, den gleichen Ausgangsstoff in der gleichen Hydrieranlage wahlweise zu einer Verbindung vom Typ I oder II, gewünschtenfalls auch zu einem Gemisch dieser Verbindungen, umzusetzen.

Die Reste $R^1$ in den Verbindungen III können im Rahmen ihrer definitionsgemäßen Bedeutung prinzipiell beliebig sein. Steht die olefinische Doppelbindung in diesen Resten in Konjugation zur

$$(-CH=\overset{|}{C}-\overset{|}{C}=O)\text{-Gruppierung}$$

so wird diese zum Teil ebenfalls hydriert, wogegen isolierte Doppelbindungen nicht angegriffen werden. Beispiele für $R^1$ sind Alkenylreste mit bis zu 20 C-Atomen und die Cyclopentenyl- und Cyclohexenylgruppe. Diese Reste können ihrerseits beispielsweise Alkylgruppen, Alkoxygruppen, Carbalkoxygruppen, Acylgruppen, Hydroxylgruppen, Carboxylgruppen, Nitrilgruppen, Aminogruppen und Halogen als Substituenten tragen. Da das Verfahrensprinzip von der Art der Substituenten $R^1$ bis $R^4$ im Rahmen ihrer definitionsgemäßen Bedeutung nicht berührt wird, erübrigt sich eine gesonderte Aufzählung der hier möglichen Ausgangsverbindungen III. Die sonstige chemische Natur im Rahmen der im Anspruch 1 gegebenen Definition spielt hier keine Rolle, jedoch handelt es sich bei III in der Praxis meist um unverzweigte oder verzweigte Alkenale und Alkenone mit bis zu 40 C-Atomen.

## Beispiel

### Herstellung von Citronellol

Jeweils 43 g reines Citral (3,7-Dimethylocta-2,6-dien-1-al) wurden in Gegenwart eines Gemisches aus einem handelsüblichen Pd/Aktivkohle-Trägerkatalysators mit 5 Gew.-% Pd-Gehalt und einem ebenfalls handelsüblichen Aktivkohle-Trägerkatalysator mit 5 Gew.-% eines der übrigen Platinmetalle hydriert. Nach Abtrennung des Katalysators wurden die Reaktionsprodukte von den hochsiedenden Rückstände abdestilliert und nach Art und Menge gaschromatographisch im Destillat bestimmt.

Die Versuchsbedingungen sowie die Verfahrensergebnisse sind der Tabelle zu entnehmen, wobei Vergleichsversuche mit »V« gekennzeichnet sind.

Tabelle

| Vers. Nr. | Katalysator Art | Gew.-Verh. | Menge g | Amin, | g | Methanol g | Druck bar | Temp. °C | Dauer h | Umsatz[1]) % | Ausbeute, %, an Citronellol | Citronellal | Geraniol/ Nerol | Dimethyloctanol[2]) | Rückstand |
|---|---|---|---|---|---|---|---|---|---|---|---|---|---|---|---|
| 1 | Pd/Ru | 1 : 9 | 1,0 | $NMe_3$[3]) | 8 | 16 | 50 | 75 | 5 | 100 | 93 | – | 4 | 1 | 1 |
| 2 | Pd/Ru | 1 : 3 | 1,0 | $NMe_3$ | 8 | 16 | 50 | 75 | 6 | 100 | 94 | 2 | 3 | – | 1 |
| 2V | Pd/Ru | 1 : 3 | 1,0 | – | – | 16 | 50 | 75 | 8 | 100 | 3 | – | 1 | 95 | 1 |
| 3 | Pd/Ru | 1 : 9 | 1,0 | $NMe_3$ | 8 | 16 | 30 | 75 | 10 | 100 | 91 | 6 | 2 | – | 1 |
| 4 | Pd/Ru | 1 : 1 | 1,0 | $NEt_3$[4]) | 8 | – | 100 | 100 | 5 | 100 | 90 | 5 | 3 | 1 | 1 |
| 5 | Pd/Ru | 1 : 3 | 1,0 | DMA[5]) | 8 | 16 | 100 | 100 | 4 | 100 | 87 | 4 | 8 | – | 1 |
| 6 | Pd/Pt | 1 : 3 | 0,1 | $NMe_3$ | 8 | 16 | 50 | 100 | 12 | 100 | 91 | 4 | 3 | 1 | 1 |
| 7 | Pd/Pt | 1 : 3 | 0,2 | $NMe_3$ | 8 | 16 | 50 | 100 | 10 | 100 | 91 | 1 | 5 | 1 | 1 |
| 8 | Pd/Ir | 1 : 3 | 1,0 | $NMe_3$ | 8 | 16 | 100 | 100 | 4 | 100 | 90 | – | – | 9 | 1 |
| 9 | Pd/Os | 1 : 9 | 1,0 | $NMe_3$ | 8 | 16 | 100 | 100 | 5 | 100 | 89 | 7 | 3 | – | 1 |
| 10 | Pd/Pt | 1 : 3 | 0,2 | $NMe_3$ | 8 | 16 | 6 | 75 | 7 | 100 | 2 | 90 | 6 | – | 1 |
| 11 | Pd/Pt[6]) | 1 : 3 | 0,2 | $NMe_3$ | 8 | 16 | 50 | 100 | 6 | 100 | 92 | 2 | 4 | 1 | 1 |

[1]) Bezogen auf Citral.
[2]) Daneben noch Dimethyloctanal $<1\%$.
[3]) Me = Methyl.
[4]) Et = Ethyl.
[5]) N,N-Dimethylanilin.
[6]) Mischkatalysator; Fällung von Pt aus $PtCl_4$-Lösung auf Pd/Aktivkohlekatalysator.

**Patentansprüche**

1. Verfahren zur Herstellung von olefinisch ungesättigten Carbonylverbindungen und Alkoholen der allgemeinen Formeln I und II

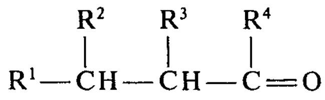
I

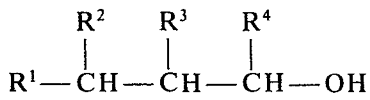
II

in denen $R^1$ einen olefinisch ungesättigten organischen Rest und $R^2$, $R^3$ und $R^4$ Wasserstoff oder eine $C_1$- bis $C_4$-Alkylgruppe bedeuten, durch Hydrierung von $\alpha,\beta$-ungesättigten Carbonylverbindungen III

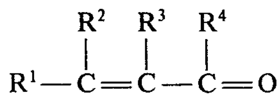
III

mit Wasserstoff in Gegenwart von Edelmetallkatalysatoren in der Flüssigphase, dadurch gekennzeichnet, daß man die Hydrierung in Gegenwart

- eines Katalysatorsystems aus Palladium einerseits und Ruthenium, Rhodium, Osmium, Iridium oder Platin als aktiven Bestandteilen andererseits sowie
- von 5 bis 40 Gew.-%, bezogen auf die Menge von III, eines tertiären Amins

vornimmt, und zwar

- zur Herstellung von überwiegenden Mengen der Verbindungen I unter einem Wasserstoffdruck von 1 bis etwa 20 bar und
- zur Herstellung von überwiegenden Mengen der Verbindungen II unter einem Wasserstoffdruck von etwa 20 bis 150 bar.

2. Verfahren nach Anspruch 1, dadurch gekennzeichnet, daß man Citral (3,7-Dimethylocta-2,6-dien-1-al, IIIa) zu Citronellal (3,7-Dimethyloct-6-en-1-al, Ia) bzw. zu Citronellol (3,7-Dimethyloct-6-en-1-ol, IIa) hydriert.

3. Verfahren nach den Ansprüchen 1 und 2,m dadurch gekennzeichnet, daß man als tertiäres Amin Trimethylamin verwendet.

**Claims**

1. A process for the preparation of olefinically unsaturated carbonyl compounds and alcohols of the general formulae I and II

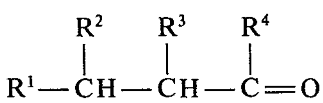
I

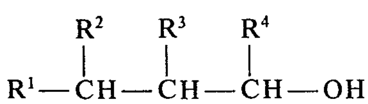
II

where $R^1$ is an olefinically unsaturated organic radical and $R^2$, $R^3$ and $R^4$ are hydrogen or $C_1-C_4$-alkyl, by hydrogenating an $\alpha,\beta$-unsaturated carbonyl compound III

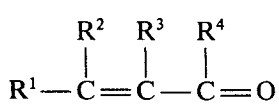
III

in the liquid phase by means of hydrogen in the presence of noble metal catalysts, wherein the hydrogenation is carried out in the presence of

a catalyst system of, palladium, on the one hand, and ruthenium, rhodium, osmium, iridium or platinum, as the active constituents, and
of from 5 to 40% by weight, based in the amount of III,
of a tertiary amine,
using a hydrogen pressure of from 1 to about 20 bar if predominantly compound I is to be obtained and
of from about 20 to 150 bar if predominantly compound II is to be obtained.

2. A process as claimed in claim 1, wherein citral (3,7-dimethylocta-2,6-dien-1-al, IIIa) ist hydrogenated to citronellal (3,7-dimethyloct-6-en-l-al, Ia) or to citronellol (3,7-dimethyloct-6-en-l-ol, IIa).
3. A process as claimed in claims 1 and 2, wherein trimethylamine is used as the tertiary amine.

**Revendications**

1. Procédé pour la préparation de composés carbonylés et d'alcools oléfiniquement insaturés de formules générales I et II

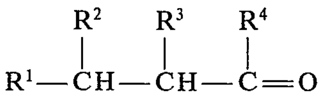

(I)

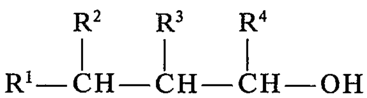

(II)

dans lesquelles $R^1$ représente un radical organique oléfiniquement insaturé et $R^2$, $R^3$ et $R^4$ représentent chacun un hydrogène ou un groupe alcoyle en $C_1$ à $C_4$, par hydrogénation de composés carbonylés $\alpha,\beta$-insaturés III

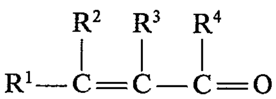

(III)

par l'hydrogène en présence de catalyseurs à base de métaux nobles en phase liquide, caractérisé en ce qu'on effectue l'hydrogénation en présence

- d'un système catalyseur composé d'une part de palladium et, d'autre part, de ruthénium, de rhodium, d'osmium, d'iridium ou de platine servant d'élément actifs, ainsi que de
- 5 à 40% en poids, par rapport à la quantité de III, d'une amine tertiaire; et ce,
- sous une pression d'hydrogène de I à environ 20 bars pour l'obtention de quantités majeures des composés I et
- sous une pression d'hydrogène d'env. 20 à 150 bars pour l'obtention de quantités majeures des composés II.

2. Procédé selon la revendication 1, caractérisé en ce qu'on hydrogène du citral (3,7-diméthylocta-2,6-diénal-I, IIIa) pour obtenir du citronellal (3,7-diméthyl-6-octènal-I, Ia) ou pour obtenir du citronellol (3,7-diméthyl-6-octènol-I, IIa).
3. Procédé selon la revendication 1 ou 2, caractérisé en ce qu'on utilise la triméthylamine en tant qu'amine tertiaire.